# EUROPEAN PATENT SPECIFICATION

(11) **EP 0 572 167 B1**
(45) Date of publication and mention of the grant of the patent: **09.10.1996**
(21) Application number: 93303891.1
(22) Date of filing: 19.05.1993
(51) Int. Cl.: A61K 7/06, A61K 7/48

(54) **Cosmetic composition**
Kosmetisches Mittel
Composition cosmétique

(30) Priority: 20.05.1992 GB 9210756
(43) Date of publication of application: 01.12.1993
(73) Proprietor: UNILEVER PLC, London EC4P 4BQ (GB); UNILEVER N.V., 3013 AL Rotterdam (NL)
(72) Inventor: Gibson, Walter Thomas, Wellingborough, Northampton NN8 6PF (GB); Westgate, Gillian Elizabeth, Irthlingborough, Northampton NN9 5RZ (GB)
(74) Representative: Mulder, Cornelis Willem Reinier, Dr.

(56) References cited:
- EP-A- 0 415 598
- DE-A- 2 025 629
- DE-B- 1 035 855
- FR-A- 1 382 068
- FR-A- 2 203 806

## Description

The invention relates to cosmetic and pharmaceutical compositions for topical application to mammalian skin or hair, containing a hair growth promoter which is capable of increasing or maintaining hair growth, especially terminal hair growth on the human scalp.

### BACKGROUND

### The Hair Growth Cycle

It should be explained that in most mammals, hair does not grow continuously, but undergoes a cycle of activity involving alternate periods of growth and rest. The hair growth cycle can be divided into three main stages, namely:
(i) the growth phase known as anagen, during which the hair follicle penetrates deep into the dermis with the cells of the bulb dividing rapidly and differentiating to form the hair,
(ii) the transitional stage known as catagen, which is heralded by the cessation of mitosis, and during which the follicle regresses upwards through the dermis and hair growth ceases,
(iii)the resting stage known as telogen, in which the regressed follicle contains a small secondary germ with an underlying ball of tightly packed dermal papilla cells.

The initiation of a new anagen phase is revealed by rapid proliferation in the germ, expansion of the dermal papilla and elaboration of basement membrane components. The hair cycle is then repeated many times until, as a consequence of the onset of male pattern baldness, most of the hair follicles spend an increasing proportion of their time in the telogen stage, and the hairs produced become finer, shorter, and less visible; this is known as terminal to vellus transformation.

### PRIOR ART

### Alleged Baldness Cures

Although there have been many claims in the scientific literature to the promotion or maintenance of hair growth by the topical application of hair tonics and the like, with the possible exception of minoxidil, none has been shown to be sufficiently free from disadvantageous clinical side effects, whether administered topically, orally or systemically, to warrant commercial exploitation as an ethical pharmaceutical, proprietary medicine, or as a cosmetic product. Possibly, the only means which has met with partial success for growing hair on the bald or balding human head is by transplantation of hair to the bald areas. This is, however, an extremely painful operation and is not always successful. Furthermore, it is immediately apparent to the casual observer that the subject has received a hair transplant and it may take many months or even years before hair regrowth, following this operation, assumes an appearance which resembles that of the original naturally growing hair.

Among the many hair regrowth studies that have been reported in the literature, there is included the work of Bazzano as described in PCT International Publication No. WO 85/04577. This publication describes a composition which is useful for increasing the rates of hair growth on mammalian skin, prolonging the anagen phase of the hair growth cycle and for treating various types of alopecias. The composition in question comprises a pyrimidine carbamate.

It has also been reported in US patent no. 4 139 619 to Chidsey assigned to the Upjohn Company, that a topical composition comprising minoxidil as the free base or acid addition salt thereof, or certain specified related iminopyrimidines, is useful in stimulating the conversion of vellus hair to growth as terminal hair, as well as increasing the rate of growth of terminal hair.

In spite of the apparent stimulation of hair growth or regrowth reported independently by Bazzano and Chidsey, following topical application of minoxidil or related compounds, there is general concern that systemic side-effects can result, particularly following topical application of minoxidil. Thus it is generally recognised in the medical literature that the side effects of orally administered minoxidil are very serious, and include fluid retention, tachycardia, dyspnea, gynecomastia, fatigue, nausea and cardiotoxicity. There is also evidence that certain side effects have been experienced following topical application of minoxidil.

It is also reported by Lion Corp., in JP 61151109 that compositions comprising mono-N-long chained acyl basic amino acid lower alkyl ester salt can, together with higher fatty acid having an odd number of carbon atoms, higher aliphatic alcohol having an odd number of carbon atoms, or their derivatives, can be used for regenerating and growth increasing effect on hair.

Finally, DE 1617477 (Fischer) discloses a hair tonic which is said to guarantee hair growth. The tonic includes a variety of amino acids as well as four vitamins.

Document FR-A-0 415 598 discloses a composition for inducing, maintaining and increasing hair growth.

### BACKGROUND TO THE INVENTION

Our own search for effective compositions that could be applied topically to the human scalp in order to promote hair growth, was influenced by the need to discover molecules which were not only effective but also completely safe in use and free from contra indications which would limit their appeal. Furthermore, we were anxious to identify relatively simple molecules in this respect which were easy to synthesis and inexpensive to deploy in a mass market affordable product which would appeal to a large number of potential consumers.

We have noted that the hair follicle has one of the highest rates of cell division in the body. This imposes considerable demands for energy to sustain rapid cell growth. Until recently, little was known of the preferred sources of energy for the hair follicle or the metabolic pathways by which they were utilised.

However, with the recent discovery of a method for maintaining follicle growth and hair production in vitro, we have been able for the first time to investigate the energy metabolism of hair follicles where we can be reasonably certain that experimental observations and conclusions will reflect the behaviour of follicles in vivo.

In the course of these experiments, we have found that the hair follicle can use, as a source of energy, several different fuels in addition to glucose. These include certain derivatives of glutamic acid.

Surprisingly, we have found that these alternative fuels do not simply act as a replacement for glucose in terms of energy production. Even in the presence of glucose, we have observed that significant stimulation of linear hair growth rate can be obtained by supplying small amounts of one or more of them.

The invention is accordingly concerned with the promotion of hair growth using special glutamic acid derivatives.

### DEFINITION OF THE INVENTION

Accordingly, the invention provides a composition suitable for topical application to mammalian skin or hair for inducing, maintaining or increasing hair growth, which comprises:
i. an effective amount of from 0.001 to 99% by weight of a hair growth promoter chosen from glutamic acid derivatives having the structure (1): 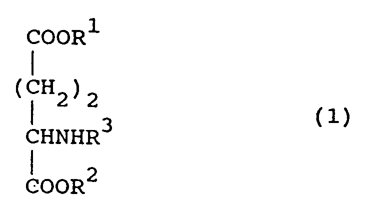 where R¹ and R² are each chosen from:
   (i) H⁺,
   (ii) alkali metal cations chosen from Na⁺, K⁺ and Li⁺,
   (iii) NH₄⁺ or alkanolammonium ions, and
   (iii) Cₓ H_{y}- ;
   the R¹ group and the R² group being the same or different;
   and where R³ is chosen from:
   (i) H-,
   (ii) Cₓ H_{y}-, and
   (iii) Cₓ H_{y} CO-;
   where x is an integer of from 1 to 22, and
   y is an integer of from 3 to 45;
   provided that at least one of the R¹ and R² groups is Cₓ H_{y}-;
   and mixtures of said glutamic acid derivatives; and
ii. from 1 to 99.99% by weight of a cosmetically acceptable vehicle for the hair growth promoter.

### DISCLOSURE OF THE INVENTION

### The hair growth promoter

According to the invention, the composition comprises a hair growth promoter chosen from glutamic acid derivatives having the Structure (1).

Preferred examples of glutamic acid derivatives having the Structure (1), where R¹ is Cₓ Hy- include:
L- glutamic acid γ-methyl ester (2)
L- glutamic acid γ-ethyl ester (3)
L- glutamic acid γ-n-propyl ester (4)
L- glutamic acid γ-isopropyl ester (5)
L- glutamic acid γ-n-butyl ester (6)
L- glutamic acid γ-n-hexyl ester (7)
L- glutamic acid γ-n-octyl ester (8)
L- glutamic acid γ-n-dodecyl ester (9)
L- glutamic acid γ-n-tetradecyl ester (10)
L- glutamic acid γ-n-hexadecyl ester (11)
L- glutamic acid γ-n-octadecyl ester (12)
and their monosodium or monopotassium salts.

Preferred examples of glutamic acid derivatives having the Structure (1), where R² is CₓH_{y}- include:
L- glutamic acid α-methyl ester (13)
L- glutamic acid α-ethyl ester (14)
L- glutamic acid α-n-propyl ester (15)
L- glutamic acid α-isopropyl ester (16)
L- glutamic acid α-n-butyl ester (17)
L- glutamic acid α-n-hexyl ester (18)
L- glutamic acid α-n-octyl ester (19)
L- glutamic acid α-n-dodecyl ester (20)
L- glutamic acid α-n-tetradecyl ester (21)
L- glutamic acid α-n-hexadecyl ester (22)
L- glutamic acid α-n-octadecyl ester (23)
and their monosodium or monopotassiam salts.

Preferred examples of glutamic acid derivatives having the Structure (1), where R³ is Cₓ H_{y}- include:
N-methyl glutamic acid (24)
N-ethyl glutamic acid (25)
N-n-propyl glutamic acid (26)
N-isopropyl glutamic acid (27)
N-n-butyl glutamic acid (28)
N-n-hexyl glutamic acid (29)
N-n-octyl glutamic acid (30)
N-n-decyl glutamic acid (31)
N-n-dodecyl glutamic acid, and (32)
N-n-octadecyl glutamic acid (33)
and their corresponding mono or di sodium and mono or di potassium salts.

Preferred examples of glutamic acid derivatives having the Structure (1), where R³ is Cₓ H_{y} CO- include:
N-acetyl glutamic acid (34)
N-n-propanoyl glutamic acid (35)
N-isopropanoyl glutamic acid (36)
N-n-butanoyl glutamic acid (37)
N-n-hexanoyl glutamic acid (38)
N-n-octanoyl glutamic acid (39)
N-n-decanoyl glutamic acid (40)
N-n-dodecanoyl glutamic acid (41)
N-n-octadecanoyl glutamic acid (42)
and their corresponding mono or di sodium salts and nono or di potassium salts.

Selected glutamic acid derivatives from those given above and identified by numbers (2) to (42) are further illustrated in the Examples given hereinafter, where they are identified as "Promoters" with the relevant numbers in parenthesis.

The composition can comprise two more hair growth promoters, as herein defined.

The total amount of the hair growth promoter present in the composition according to the invention is an amount which is sufficient to induce maintain or increase hair growth. This amount will depend on the effectiveness of the promoter, some being more effective than others, but in general an amount of from 0.001 to 99%, usually from 0.01 to 20% by weight of the composition will provide an adequate concentration for application to the skin, particularly the scalp, which can then be repeated as necessary to promote hair growth.

### The Cosmetically Acceptable Vehicle

The composition according to the invention also comprises a solid, semi-solid or liquid cosmetically and/or physiologically acceptable vehicle, to enable the hair growth promoter to be conveyed to the skin at an appropriate dilution. The nature of the vehicle will depend upon the method chosen for topical administration of the composition. The vehicle can itself be inert or it can possess physiological or pharmaceutical benefits of its own.

The selection of a vehicle for this purpose presents a wide range of possibilities depending on the required product form of the composition. Suitable vehicles can be classified as described hereinafter.

It should be explained that vehicles are substances which can act as diluents, dispersants, or solvents for the hair growth promoter which therefore ensure that they can be applied to and distributed evenly over the hair and/or scalp at an appropriate concentration. The vehicle is preferably one which can aid penetration of the esters into the skin to reach the immediate environment of the hair follicle. Compositions according to this invention can include water as a vehicle, and/or at least one cosmetically acceptable vehicle other than water.

Vehicles other than water that can be used in compositions according to the invention can include solids or liquids such as emollients, solvents, humectants, thickeners and powders. Examples of each of these types of vehicles, which can be used singly or as mixtures of one or more vehicles, are as follows:
Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polythylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;
Propellants, such as propane, butane, isobutane, dimethyl ether, carbon dioxide, nitrous oxide;
Solvents, such as ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide, tetrahydrofuran;
Humectants, such as glycerin, sorbitol, sodium 2-pyrrolidone-5-carboxylate, soluble collagen, dibutyl phthalate, gelatin;
Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The amount of vehicle in the composition, including water if present, should preferably be sufficient to carry at least a portion of a selected hair growth promoter to the skin in an amount which is sufficient effectively to enhance hair growth. The amount of the vehicle can comprise the balance of the composition, particularly where little or no other ingredients are present in the composition. Accordingly, the vehicle or vehicles can comprise from 1 to 99.99%, preferably from 50 to 99.5% and ideally from 90 to 99% by weight of the composition.

### Perfume

The composition according to the invention can also optionally comprise a perfume in an amount sufficient to make the composition acceptable to the consumer and pleasant to use. Usually, the perfume will form from 0.01 to 10% by weight of the composition.

### Activity Enhancer

The composition according to the invention can also optionally comprise an activity enhancer.

The activity enhancer can be chosen from a wide variety of molecules which can function in different ways to enhance the hair growth effects of the hair growth promoter. Particular classes of activity enhancers include (a) other hair growth stimulants, (b) penetration enhancers and (c) cationic polymers, whose presence can further improve the delivery of the ester through the stratum corneum to its site of action in the immediate environment of the hair follicle.

Some activity enhancers can also function as vehicles for the ester.

### (a) Other Hair Growth Stimulants

i. Examples of other substances which themselves possess the ability to stimulate or increase hair growth include, for example:
   Benzalkonium chloride
   Benzethonium chloride
   Phenol
   Estradiol
   Diphenhydramine hydrochloride
   Chlorpheniramine maleate
   Chlorophyllin derivatives
   Cholesterol
   Salicylic acid
   Cystine
   Methionine
   Red pepper tincture
   Benzyl nicotinate
   dl-Menthol
   Peppermint oil
   Calcium pantothenate
   Panthenol
   Castor oil
   Hinokitiol
   Prednisolone
   Resorcinol
   Further substances which themselves possess the ability to increase the rate of terminal hair growth include:
ii. α-1,4 esterified disaccharides described by Choay S.A. in EP-A-O 064 012, having the structure (3): 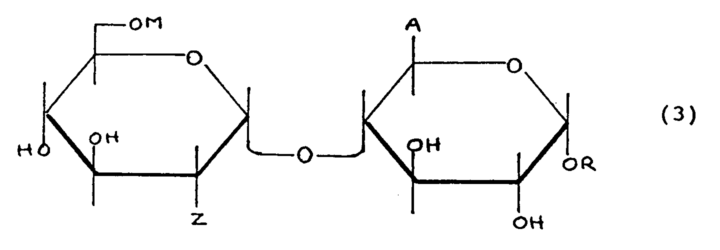
   - where: Z represents a functional nitrogen group, such as an azide or a group having the structure -NHB, in which B represents -H or a functional group such as acetyl or sulphate as a salt with an organic or mineral cation;
   M represents -H or SO₃M₁, where M₁ is an organic or metallic cation, particularly an alkali metal; or an acetyl group;
   R represents a C₁ to C₄ alkyl radical, especially methyl; or an aryl radical;
   A represents a functional group such as an acid or -COOR₁, where R₁ represents -H or a C₁ to C₄ alkyl radical, especially methyl; or a metal, especially an alkali metal;
   esterified oligosaccharides as described by Unilever in EP-A-O 211 610, including at least one esterified disaccharide unit consisting of a uronic acid residue having the structure (4): 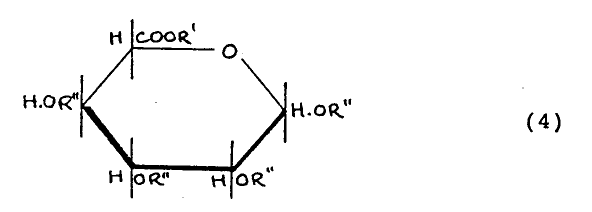 and a hexosamine residue having the structure (5): 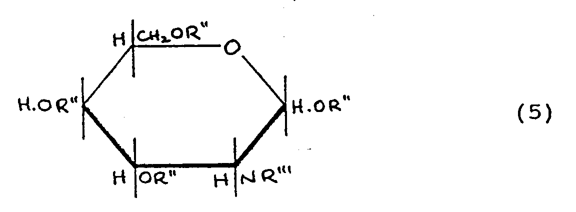
   - where: R' is -H, C₃ to C₁₀ alkyl or 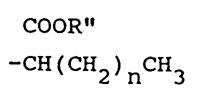 R'' is -H, C₁ to C₄ alkyl, -CO(CH₂)ₘCH₃, -SO₃M,
   R''' is -H, -CO(CH₂)ₘCH₃, or -SO₃M,
   M is -H, or a metallic or organic cation
   n is 0 or an integer of from 1 to 7, and
   m is 0 or the integer 1 or 2;
   the groups designated R'' being the same or different, one R'' group from each pyranose ring structure being linked by a glycosidic linkage having the configuration α-1,3, α-1,4, β-1,3 or β-1,4; and the -COOR', -CH₂OR'' and -OR'' groups being of either configuration with respect to the pyranose rings;
iii. Minoxidil glucuronides, as described by Unilever in EP-O 242 967,
iv. Minoxidil sulphates, as described by The Upjohn Co. in WO 86/04231, and
v. Minoxidil, and other derivatives thereof as described by The Upjohn Co, in US patent 4 139 619.
vi. Ethylenediaminetetraacetic acid or salts thereof, as described by Redken Laboratories, Inc. in US 4 814 351.
vii. Direct proteoglycanase inhibitors, such as 1,10-phenanthroline, as described by Unilever in EP-0 277 428.
viii.Glycosaminoglycanase inhibitors, as described by Unilever in EP-0 277 428, such as aldonolactones and esterified aldonolactones,
   preferred examples of which include:
   L-Galactono-1,4-lactone
   L-Arabino-1,5-lactone
   D-Fucono-1,5-lactone
   D-Glucaro-1,4-lactone
   D-Glucurono-6,3-lactone
   Galactaric acid lactone
   2-Acetamido-2-deoxygluconolactone
   2-Acetamido-2-deoxygalactono-lactone
   D-Glucaro-1,4:6,3-dilactone
   L-Idaro-1,4-lactone
   2,3,5-Tri-0-acetyl-D-glucaro-1,4-lactone
   2,5-Di-0-acetyl-D-glucaro-1,4:6,3-dilactone.
ix. Glycosaminoglycanase inhibitors, as described by Unilever in EP 0 277 428, such as monosaccharides and esterified monosaccharides,
   preferred examples of which include:
   N-Acetylglucosamine
   N-Acetylgalactosamine
   D-Galactosamine
   D-Glucosamine-3-sulphate
   N-Acetylmannosamine.
x. Glycosaminoglycan chain cellular uptake inhibitors, as described by Unilever in EP 0 277 428, such as hexuronic acid and esters thereof.
xi. Chemical inhibitors of glycosidase activity, as described by Unilever in EP 0 334 586, chosen from lactams,
   preferred examples of which include:
   D-glucaro-1,5-lactam,
   L-Galactono-1,4-lactam,
   L-Arabino-1,5-lactam,
   D-Fucono-1,5-lactam,
   D-Glucaro-1,4-lactam,
   D-Glucurono-6,3-lactam,
   1,2,5-tri-O-acetyl-D-glucurono-6,3-lactam,
   2-Acetamido-2-deoxygluconolactam,
   2-Acetamido-2-deoxygalactonolactam,
   D-Glucaro-1,4:6,3-dilactam,
   L-Idaro-1,4-lactam,
   2,3,5-Tri-O-acetyl-D-glucaro-1,4-lactam,
   2,5-Di-O-acetyl-D-Glucaro-1,4:6,3-dilactam,
   D-glucaro-1,5-lactam ethyl ester;
xii. Chemical activators of protein kinase C enzymes, as described by Unilever in EP 0 334 585 chosen from diacylglycerols,
   preferred examples of which include:
   1,2-Dibutanoyl-rac-glycerol
   1,2-Dihexanoyl-sn-glycerol
   1,2-Dioctanoyl-rac-glycerol
   1,2-Dioctanoyl-sn-glycerol
   1,2-Didecanoyl-rac-glycerol
   1-Oleoyl-2-acetyl-rac-glycerol
   1-Oleoyl-2-acetyl-sn-glycerol
   1-Stearoyl-2-arachidonoyl-sn-glycerol
   1,2-Distearoyl-rac-glycerol
   1,2-Dipentadecanoyl-sn-glycerol
   1,2-dipentadecanoyl-rac-glycerol
   1,2-Dipalmitoyl-rac-glycerol
   1,2-Dipalmitoyl-sn-glycerol
   1,2-Diseptadecanoyl-rac-glycerol
   1,2-Dioleoyl-sn-glycerol
   1,2-Dioleoyl-rac-glycerol
   1,2-Diarachidonoyl-sn-glycerol
   1,2-Dieicosanoyl-sn-glycerol
   1,2-Didoeicosanoyl-rac-glycerol, and
   1,2-Dioctaeicosanoyl-sn-glycerol.
xiii.Glycosaminoglycanase inhibitors, as described by Unilever in EP 0 348 184, chosen from aldonomonolactone or alduronomonolactone derivatives,
   preferred examples of which aldonomonolactone derivatives include:
   6-acetyl-galactono-1,4-lactone
   6-propionyl-galactono-1,4-lactone
   6-butyryl-galactono-1,4-lactone
   2-propionamido-2-deoxygluconolactone
   2-butyramido-2-deoxygluconolactone
   2-propionamido-2-deoxygalactonolactone
   2-butyramido-2-deoxygalactonolactone
   6-propionyl-2-acetamido-2-deoxygluconolactone
   diacetyl-6-propionyl-2-acetamido-2-deoxygluconolactone
   6-butyryl-2-acetamido-2-deoxygalactonolactone diacetyl-6-butyryl-2-acetamido-2-deoxygalactonolactone
   2,3,5,6-tetraacetyl-galactono-1,4-lactone
   2,3,5-triacetyl-6-propionylgalactono-1,4-lactone
   triacetyl-2-propionamido-2-deoxygalactonolactone
   triacetyl-2-butyramido-2-deoxygluconolactone
   6-methyl-glucaro-1,4-lactone
   2,3,5,6-tetramethyl-glucaro-1,4-lactone
   6-methyl-2,3,5-triacetylglucaro-1,4-lactone
   6-methyl-3-methyl-glucaro-1,4-lactone, and
   6-methyl-3-acetyl-glucaro-1,4-lactone;
   and a preferred example of which alduronomonolactone derivative is:
   1,2,5-triacetyl-glucurono-6,3-lactone.
xiv. Glycosaminoglycanase inhibitors, as described by Unilever in EP 0 348 184, chosen from acylated monosaccharides,
   preferred examples of which acylated monosaccharides include:
   2-propionamido-2-deoxyglucose
   1,3,4,6-tetraacetyl-2-propionamido-2-deoxyglucose
   2-butyramido-2-deoxygalactose
   1,3,4,6-tetraacetyl-2-butyramido-2-deoxygalactose
   2-sulphamido-2-deoxygalactose
   2-sulphamido-2-deoxyglucose
   2-butyramido-2-deoxymannose
   1,3,4,6-tetraacetyl-2-butyramido-2-deoxymannose
   2-butyramido-2-deoxyglucose, and
   1,3,4,6-tetraacetyl-2-butyramido-2-deoxyglucose.
xv. Esters of pyroglutamic acid, as described by Lever Brothers Company in US patent No. 4 774 255,
   preferred examples of which include:
   pyroglutamic acid methyl ester
   pyroglutamic acid ethyl ester
   pyroglutamic acid n-propyl ester
   pyroglutamic acid n-butyl ester
   pyroglutamic acid n-hexyl ester
   pyroglutamic acid n-heptyl ester
   pyroglutamic acid n-octyl ester
   pyroglutamic acid n-nonyl ester
   pyroglutamic acid n-decyl ester
   pyroglutamic acid n-undecyl ester
   pyroglutamic acid n-dodecyl ester
   pyroglutamic acid n-tridecyl ester
   pyroglutamic acid n-tetradcyl ester
   pyroglutamic acid n-hexadecyl ester
   pyroglutamic acid n-octadecyl ester
   pyroglutamic acid n-eicosyl ester
   pyroglutamic acid iso-propyl ester
   pyroglutamic acid 2-methylhexyl ester
   pyroglutamic acid 2-ethylhexyl ester
   pyroglutamic acid 3,7-dimethyloctyl ester
   pyroglutamic acid 2-hexyldecyl ester
   pyroglutamic acid 2-octyldodecyl ester
   pyroglutamic acid 2,4,4-trimetyl-1-pentane ester
   pyroglutamic acid methyloctyl ester
   2-[pyroglutamoyloxy]-propionic acid
   methyl-2-[pyroglutamoyloxy]-acetate
   ethyl-2-[pyroglutamoyloxy]-n-propionate
   ethyl-2-[pyroglutamoyloxy]-n-butyrate
   ethyl-2-[pyroglutamoyloxy]-iso-butyrate
   ethyl-2-[pyroglutamoyloxy]-n-valerate
   ethyl-2-[pyroglutamoyloxy]-n-caproate
   ethyl-2-[Pyroglutamoyloxy]-n-heptylate
   ethyl-2-[pyroglutamoyloxy]-n-caprylate
   ethyl-2-[pyroglutamoyloxy]-n-pelargonate
   ethyl-2-[pyroglutamoyloxy]-3-hydroxybutyrate
   iso-propyl-2-[pyroglutamoyloxy]-n-propionate
   iso-propyl-2-[pyroglutamoyloxy]-n-caprylate
   n-propyl-2-[pyroglutamoyloxy]-n-propionate
   n-propyl-2-[pyroglutamoyloxy]-n-caprylate
   stearyl-2-[pyroglutamoyloxy]-n-propionate
   12-hydroxystearyl-2-[pyroglutamoyloxy]-n-propionate
   stearyl-2-[pyroglutamoyloxy]-n-stearate
   palmityl-2-[pyroglutamoyloxy]-n-propionate
   linoleyl-2-[pyroglutamoyloxy]-n-propionate
   linoleyl-2-[pyroglutamoyloxy]-n-caprylate
   lauryl-2-[pyroglutamoyloxy]-n-caprylate
   stearyl-2-[pyroglutamoyloxy]-n-caprylate
   glyceryl mono(2-[pyroglutamoyloxy]-n-propionate)
   glyceryl mono(2-[pyroglutamoyloxy]-n-caprylate), and
   glyceryl di(2-[pyroglutamoyloxy]-n-propionate).
xvi. hexosaccharic acids or an acylated hexosaccharic acids, or salts or esters thereof, as described by Unilever in EP 378 388
   preferred examples of which include:
   allosaccharic acid
   altrosaccharic acid
   glucosaccharic acid
   mannosaccharic acid
   gulosaccharic acid
   idosaccharic acid
   galactosaccharic acid
   talosaccharic acid, and
   their disodium salts.
xvii.aryl-substituted ethylenes as described by Unilever in EP 403 238,
   preferred examples of which include:
   1-carboxy-2-(4-hydroxyphenyl)ethylene
   1,1-dicarboxy-2-(4-hydroxyphenyl)ethylene
   1,1-dicyano-2-(4-hydroxyphenyl)ethylene
   1-carboxy-2-(3,4-dihydroxyphenyl)ethylene
   1,1-dicyano-2-(3-hydroxyphenyl)ethylene
   1-cyano-1-carboxy-2-(2,5-dihydroxyphenyl)ethylene
   1-carboxy-1-cyano-2-(3,4-dihydroxyphenyl)ethylene
   1,1-dicyano-2-(3,4-dihydroxyphenyl)ethylene
   1,1-dicyano-2-(3-methoxy-4,5-dihydroxyphenyl)ethylene
   1,1-dicyano-2-(3,4,5-trihydroxyphenyl)ethylene
   1-amido-1-cyano-2-(3,4-dihydroxyphenyl)ethylene
   1-thioamido-1-cyano-2-(3,4-dihydroxyphenyl)ethylene
   1-cyano-2-(4-hydroxyphenyl)ethylene
   1,1-dicyano-2-(3-hydroxy-4-nitrophenyl)ethylene
   1,1-dicyano-2-hydroxy-2-(4-hydroxyphenyl)ethylene
   1,1-dicyano-2-(3-methoxy-4-hydroxyphenyl)ethylene
   1,1-dicyano-2-(3,5-dihydroxyphenyl)ethylene
   1,1-dicyano-2-hydroxy-2-(3,4,5-trihydroxyphenyl)ethylene
   1-carboxy-1-cyano-2-(4-methoxyphenyl)ethylene
   1-carboxy-1-cyano-2-(4-fluorophenyl)ethylene
   1-carboxy-1-cyano-2-(3-methoxy-4-hydroxyphenyl)ethylene
   1-carboxy-1-cyano-2-(3,5-dimethoxy-4-hydroxyphenyl)ethylene
   1-carboxy-1-cyano-2-(4-hydroxyphenyl)ethylene
   1-carboxy-1-cyano-2-(4-phenylcarboxyaldehyde)ethylene, and
   1-cyano-1-carboxy-2-(2,5-dihydroxyphenyl)ethylene
xviii. N-acylated amino acids as described by Unilever in EP 415 598.
   Preferred examples of which include:
   N-acetyl glycine
   N-acetyl hydroxyproline
   N-acetyl alanine
   N-acetyl valine
   N-acetyl leucine
   N-acetyl isoleucine
   N-acetyl phenylalanine
   N-acetyl tyrosine
   N-acetyl proline
   N-acetyl serine
   N-acetyl threonine
   N-acetyl cysteine
   N-acetyl cystine
   N-acetyl methionine
   N-acetyl tryptophan
   N-lauroyl glycine
   N-palmitoyl glycine
   N-myristoyl glycine
   N-lauroyl hydroxyproline
   N-octanoyl glycine
   N-octanoyl hydroxyproline
   N-hexanoyl glycine
   N-acetyl aspartic acid
   N-lauroyl aspartic acid
   N-palmitoyl aspartic acid
   N-octanoyl aspartic acid
   N-acetyl glutamic acid
   N-lauroyl glutamic acid
   N-palmitoyl glutamic acid
   N-octanoyl glutamic acid
   N-acetyl arginine
   N-acetyl lysine
   N-acetyl histidine
   N-acetyl ornithine
   N-acetyl hydroxylysine
   N-acetyl citrulline
   N-lauroyl lysine
   N-lauroyl citrulline
   N-myristoyl citrulline
   N-myristoyl ornithine
   N-octanoyl lysine, and
   N-octanoyl citrulline.

### (b) Penetration Enhancers

As has been stated earlier, the presence of a penetration enhancer can potentiate the benefit of the hair growth promoter by improving its delivery through the stratum corneum to its site of action in the immediate environment of the hair follicle close to the dermal papilla.

The penetration enhancer can accordingly function in a variety of ways. It can for example, improve the distribution of the hair growth promoter on the skin surface or, it can increase its partition into the skin from the composition when applied topically, so aiding its passage to its site of action. Other mechanisms enhancing the benefit of the hair growth promoter may also be involved.
Examples of penetration enhancers include:
2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-hydroxypropanoic acid
2-hyroxyoctanoic acid,
Dimethyl sulphoxide
N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl-2-pyrrolidone
Phosphine oxides
Sugar esters
Tetrahydrofurfural alcohol
Urea
Diethyl-m-toluamide, and
1-Dodecylazacyloheptan-2-one

### (c) Cationic Polymers

As stated earlier, the presence of a cationic polymer can potentiate the benefit of the hair growth promoter by improving its delivery to the hair and scalp. Examples of preferred cationic polymers include:
Guar Hydroxypropyltrimonium chloride
Quaternium-19
Quaternium-23
Quaternium-40
Quaternium-57
Poly(dipropyldiallylammonium chloride)
Poly(methyl-γ-propaniodiallylammonium chloride)
Poly(diallylpiperidinium chloride)
Poly(vinyl pyridinium chloride)
Quaternised poly (vinyl alcohol)
Quaternised poly (dimethylaminoethylmethacrylate); and
mixtures thereof

The amount of activity enhancer, when employed in accordance with the invention, will normally be from 0.1 to 50%, preferably from 0.5 to 25% and most preferably from 0.5 to 10% by weight of the composition.

### Other hair growth promoter adjuncts

The composition according to the invention can also contain adjuncts other than those already mentioned, depending on the form of the intended product. It is, for example, possible to include antiseptics, preservatives, antioxidants, emulsifiers and colouring agents, pearlescers, foam boosters, conditioning agents (such as cationic surfactants, cationic polymers and silicones) and agents such as PFPE (perfluoropolyethylene) for improving hair gloss, which can improve the stability and consumer appeal of the composition.

The composition according to the invention can also be employed as a vehicle for a wide variety of cosmetically or pharmaceutically active ingredients, particularly ingredients which have some beneficial effect other than the promotion of hair growth when applied to the skin.

### Surfactants

The composition for use in the method according to the invention can be formulated as a shampoo and will then accordingly comprise one or more surfactants which are cosmetically acceptable and suitable for topical application to-the hair. Examples of suitable shampoo surfactants are now given.

### Anionic surfactant

The composition of the invention can comprise an anionic surfactant which is preferably chosen from alkyl sulphate, alkyl ether sulphate, alkyl sulphonate, alkyl aryl sulphonate, olefin sulphonate, acyl sarcosinate, acyl tauride, acyl isethionate, nonoalkyl sulphosuccinate, dialkylsulphosuccinate, acryl lactylate, acylated α-amino acid, allky carboxylate, monoalkyl phosphate and dialkyl phosphate.

Specific examples of anionic surfactants include:
alkyl sulphates, such as sodium lauryl sulphate [eg. EMPICOL CX available from Albright & Wilson], and triethanolaminde lauryl sulphate [eg. EMPICOL TL40/T, available from Albright & Wilson].
alkylether sulphates, such as sodium lauryl ether sulphate [eg. EMPICOL ESB70, available from Albright & Wilson].
alkyl sulphonates, such as sodium alkane (C₁₃₋₁₈) sulphonate [eg. HOSTAPUR SAS 30, available from Hoechst].
alkylaryl sulphonates, such as sodium alkyl benzene sulphonate [eg. TEEPOL CM44, available from Shell].
olefin sulphonates, such as sodium olefin sulphonate (C₅₋₁₈) [eg. HOSTAPUR OS, available from Hoechst].
acyl sarcosinates, having the structure: (51) 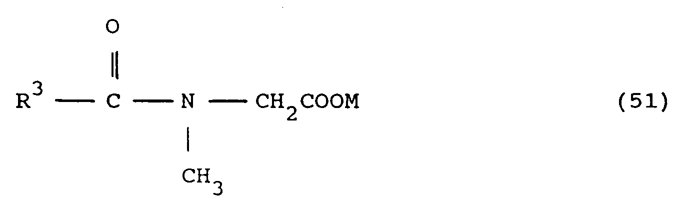
- where: R³ is chosen from C₆₋₁₄ alkyl, and
M is a counterion chosen from alkali metals, ammonium and substituted ammonium such as alkanolammonium.

An example of an acyl sarcosinate having the structure (51), is sodium laurly sarcosinate [eg. HAMPOSYL L-95, available from Grace].
acyl taurides, having the structure (52): 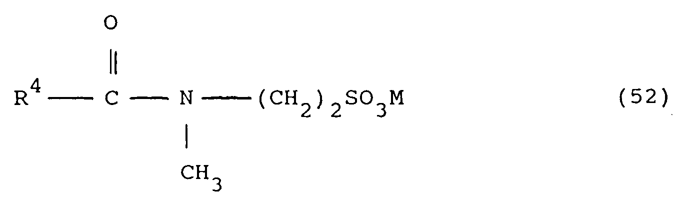
where R⁴ is chosen from C₈₋₁₈ alkyl

An example of an acyl tauride having the structure (52) is coconut methyl taurine [eg. FENOPEN TC 42, available from GAF].
acyl isethionates, having the structure (53): 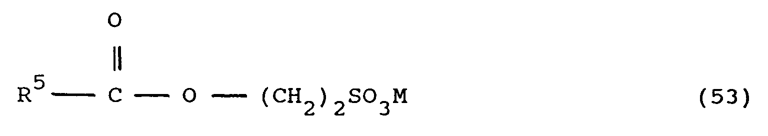
where R⁵ is chosen from C₈₋₁₈ alkyl.

An example of an acyl isethionate having the structure (53) is sodium acyl isethionate [eg. JORDAPON C1, available from Jordon].
monoalkyl sulphosuccinates, having the structure (54): 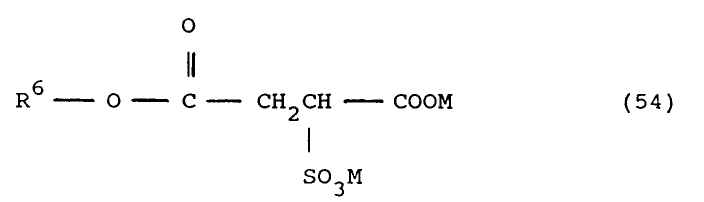
where R⁶ is chosen from C₁₀₋₂₀ alkyl.

Examples of monoalkyl sulphosuccinates having the structure (54) include:
sodium lauryl sulphosuccinate [eg. EMPICOL SLL, available from Albright & Wilson].
magnesium alkyl sulphosuccinate [eg. ELFANOL 616 Mg, available from AKZO].
sodium lauryl ethoxysulphosuccinate [eg. EMPICOL SDD, available from Albright & Wilson].
coconut monoethanolamide ethoxysulphosuccinate [eg. EMPICOL SGG].
disodium lauryl polyglycolether sulphosuccinate [eg. SURTAGENE S30, available from CHEM-Y].
polyethyleneglycol sulphosuccinate [eg. REWOPOL SBFA 30, available from REWO].
dialkyl sulphosuccinates, having the structure (55): 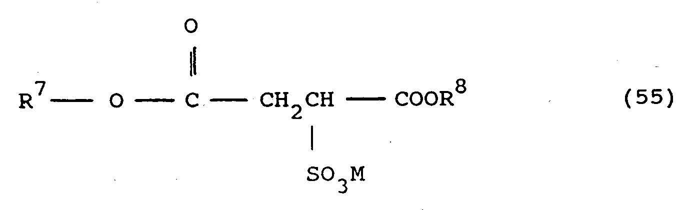
where R⁷ and R⁸ are the same or different, and are chosen from C₆₋₁₄ alkyl.

An example of a dialkyl sulphosuccinate having the structure (55) is sodium dilauryl sulphosuccinate [eg. EMCOL 4500, available from Witco].
acyl lactylates, having the structure (56): 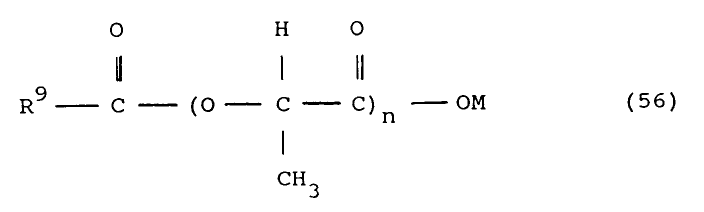
where R₉ is chosen from C₆₋₁₆ alkyl,
and n is 1 or 2.

An example of an acyl lactylate having the structure (6) is decanoyl lactylate [eg. PATIONIC 122a, available from Patterson, CJ].
acylated α-amino acids, such as sodium lauroyl glutamate [eg. ACYLGLUTAMATE LS-11, available from Ajinomoto Co. Inc].
ethyl carboxlates, such as alkyl
C₁₂₋₁₄O(EO)₄OCH₂CO₂Na [eg. AKYPO RLM 38, available from Akzo].
monoalkyl phosphates and dialkyl phosphates, such as dioctyl phosphate.

### Amphoteric surfactant

The shampoo compositions of the invention also comprise amphoteric surfactant. Suitable amphoteric surfactants are derivatives of aliphatic quaternary ammonium, phosphonium and sulphonium compunds, wherein the aliphatic radicals contain from 8 to 18 carbon atoms, and may be straight chain or branched, and further contain an anionic water-solubilising group, such as carboxyl, sulphonate, sulphate, phosphate or phosphonate.

Preferred amphoteric surfactants include:
Alkyl betaines, having the structure (57): 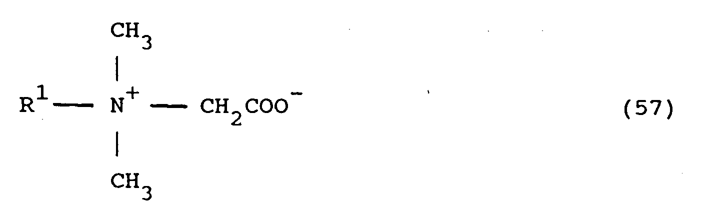
where R¹ is C₁₋₁₆ alkyl.

An example of an alkyl betaine having the structure (7) is lauryldimethyl betaine [eg. EMPIGEN BB, available from Albright & Wilson].
Alkylamidopropyl betines, having the structure (58):

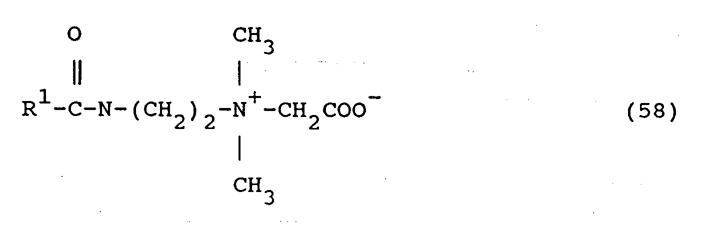

An example of an alkylamidopropyl betaine having the structure (58) is cocamidopropy betaine [eg. TEGOBETAIN L7, available from Goldschmidt).
Alkylamphoglycinates or Alkylamphopropionates having the structure (59): 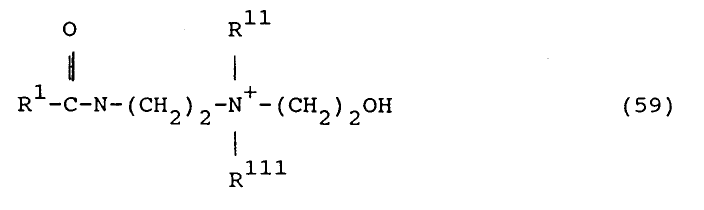
where R¹¹ is chosen from H, CH₂COO⁻ and (CH₂)₂COO⁻, and
R¹¹¹ is chosen from CH₂COO⁻ and (CH₂)₂COO⁻

Suitable examples of compounds (59) are cocoamphoglycinate (available from GAF), and cocoamphopropionate.
Sultaines, having the structure (60): 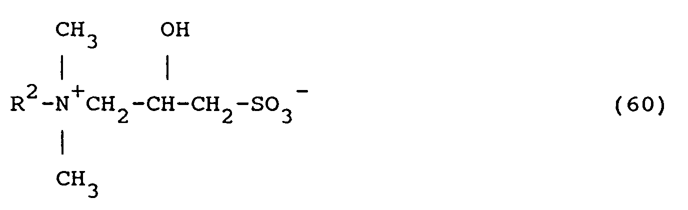
where R² is chosen from C₁₂₋₁₆ alkyl alkylamido groups.

An example of a sultaine having the structure (60) is cocamidopropylhydroxysultaine [eg. CYCLOTERIC BET-CS, available from Alcolac).

The most preferred amphoteric surfactant are lauryl dimethyl betaine and cocamidopropyl betaine.

Such amphoteric surfactants can contribute to the foaming of the shampoo of the invention, while ameliorating the harshness of the anionic surfactant.

### Nonionic surfactant

The shampoo composition of the invention can also comprise alkoxylated or glycosidic nonionic surfactant having an HLB of 8 or more. Above this value nonionics generally form clear isotropic solutions in combination with the other surfactants in the ranges defined above. Preferred nonionic surfactants are polyoxyethylene alkyl esters and polyoxyethylene alkyl ethers and alkyl polyglycosides.

A suitable example of a polyoxyethylene alkyl ester is that having the CTFA designation Polysorbate 80 which is a mixture of oleate esters of sorbitol and sorbitol anhydrides, condensed with approximately 20 moles of ethylene oxide. Also suitable is Polysorbate 20 which is a mixture of laurate esters or sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of C₉₋₁₁ alcohol with an average of 8 ethoxy units, which is available commerically as NONIDET LE-8T or as SYNPERONIC 91-8T, and the polyethylene glycol ether of C₁₂₋₁₅ alcohol with an average of 9 ethoxy units which is available commerically as DOBANOL 25-9.

Particularly useful alkyl polyglycosides include the glycosides of glucose or glucose oligomers where the alkyl chain can be C₈₋₁₆ and the average number of glucose units is 1 to 2. A suitable example is ORAMIX NS 10 which is the glucoside of C₁₀₋₁₂ fatty alcohol with an average of about 1.5 glucose units.

The amount of surfactant that can be present in the composition accordingly to the invention is up to 30%, preferably from 1 to 20% by weight of the composition.

### Preservation of the Composition

The composition according to the invention is preferably preserved in such a manner that it will enjoy an extended shelf life following manufacture and prior to sale and use. Ideally the composition will have an indefinite shelf life.

It is accordingly apparent that the hair growth promoter is likely to be prone to attack by bacteria, moulds and fungi and other microbial influences, particularly at pH values near that of the skin that characterise the preferred composition. The shelf-life of the composition can therefore be unacceptably short due to the biodegradation of the hair growth promoter unless steps are taken to preserve the composition.

In order to be preserved, the composition should preferably be free, or substantially free, from viable microbial contaminants that are capable of resulting in microbial spoilage of the composition, and/or biodegradation of the hair growth promoter prior to topical application of the composition to mammalian skin or hair. It is to be understood, however, that the invention is also concerned with compositions, as herein defined, which may contain viable but dormant microorganisms, such as bacterial spores, provided that the conditions of preservation do not result in substantial proliferation of the microorganisms prior to use of the composition.

Examples of methods that can be employed to achieve preservation of the composition, includes the following:

### (i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

### (ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

### (iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to the invention to reduce the water activity (α_{w}) from 1 to < 0.9, preferably to < 0.85 and most preferably < 0.8, the lowest of these values being that at which yeasts, moulds and fungi will not proliferate.

### pH

The hair growth promoter may be susceptable to hydrolysis, particularly when the pH value of the composition is alkaline. It is accordingly preferred that the composition, when aqueous, should have an acid pH value. The preferred pH value of the composition, when aqueous, is from 2 to <7, ideally from 4 to 6.5.

### Process

The invention also provides a process for the preparation of a composition suitable for topical application to mammalian skin or hair which comprises mixing a hair growth promoter as herein defined, with a suitable vehicle to provide a composition according to the invention, in which the hair growth promoter forms from 0.0001 to 99% by weight of the composition.

### Product Form and Container

The compositions of the invention can be formulated as liquids, for example as a lotion, shampoo, conditioner, milk or cream for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example sticks, creams or gels, for use in conjunction with a suitable applicator or simply a tube, bottle or lidded jar, or as a liquid-impregnated fabric, such as a tissue wipe.

The invention accordingly also provides a closed container containing a composition as herein defined.

### Use of the hair growth promoter

The invention also provides for the use of hair growth promoter as herein defined, for topical application to mammalian skin or hair for inducing, maintaining or increasing hair growth.

The compositions according to the invention are primarily intended for topical application to the scalp of the human subject, particularly where the head is already bald or balding, in order to convert vellus hair to growth as terminal hair, or to increase the rate of growth of terminal hair. The compositions can also be applied profilactically to the hair and hence the scalp to reduce or prevent the onset of baldness.

The amount of the composition and the frequency of application to the hair and/or scalp can vary widely, depending on personal needs, but it is suggested as an example that topical application of from 0.1 to 5g daily containing from 0.00001 to 1g of a selected chemical inhibitor over the period of at least six months will in most cases result in an improvement in hair growth.

### EVALUATION OF EFFICACY OF THE HAIR GROWTH PROMOTERS USING THE IN VITRO HAIR FOLLICLE GROWTH TEST

The effect of compounds on hair growth was assessed using an in vitro test which measures the elongation of isolated human hair follicles in a culture medium.

### Isolation of the hair follicle from skin

This test includes the important step of isolating hair follicles having an undamaged hair bulb from human skin, for example, facelift skin, by microdissection.

The critical step of separating the hair follicle with intact undamaged hair bulb from the subcutaneous fatty tissue in which it is situated accordingly involves severing the hair shaft of the follicle at a point below the epidermis of skin surface, so as to leave the hair bulb intact and undamaged while still bearing a portion of the hair shaft.

Preferably, the hair shaft of the follicle is severed at the dermal-subcutaneous fat interface.

Any suitable cutting instrument can be employed to sever the hair shaft in this manner, but a keratotome or a scalpel are preferred.

The hair bulb with a hair shaft stump attached is then isolated from the skin by mechanically separating the hair from loosely adhering subcutaneous fat which normally surrounds the hair bulb. This is achieved after the dermis or upper layer of the skin has been separated and removed, to avoid damaging the hair bulb as it is pulled away.

The hair bulb together with hair shaft stump attached, is then transferred in an otherwise undamaged and fully functioning, viable state to a nutrient medium.

### Culture of the isolated hair follicle

The hair follicles isolated by the technique described herein are transferred to a suitable culture medium for subsequent testing of substances that can then influence their future development.

The procedure now to be described represents a preferred method of culture and testing of hair growth.

In accordance with the preferred method of culture, isolated hair follicles, obtained from facelift skin from a 61 year old female, are maintained in 1 ml of Williams E medium, either with or without a test hair growth substance, supplemented with antibodies (Penicillin and Streptomyein), Insulin (10ng/ml) and Hydrocortisone (10ng/ml). The medium was incubated at 37°C in an atmosphere of 5% CO₂ + 95% air in individual wells of a 24 multiwell dish (Corning), which permits detailed measurements to be made of the length of individual hair follicles. The medium was refreshed once during the experiment after 4 days.

Williams E medium is available from FLOW Laboratory under Catalogue No. 12-502. The formula of Williams E medium is described by Williams GM, et al., in Experimental Cell Research 69 (1971) on page 106.

Daily growth rate and cumulative growth for each follicle were calculated by measuring the change in length of the follicles each day and from this the average of all the follicles was calculated.

### Evaluation of Results

The response of an isolated hair follicle to a test substance, can accordingly be assessed by measuring the increase in length, if any, in the presence of a test substance against a control.

The in vitro method described herein was used to assess the effect of two 'fuels' (hair growth promoters), namely a glutamic acid derivative, as herein defined, and glutamine on hair growth.

### EXAMPLES

The invention is illustrated by the following examples.

### Example 1

This Example illustrates a lotion according to the invention which is suitable for topical application to the scalp in order to promote hair growth.

The lotion has the following formulation:

| | % w/w |
|---|---|
| Promoter No. (2) | 1 |
| ethanol | 99 |
| perfume | q.s. |

### Example 2

This Example illustrates a hair tonic which is suitable for application to hair or scalp.

The hair tonic has the following formulation:

| | % w/w |
|---|---|
| Promter No. (22) | 2 |
| ethanol | 49 |
| water | 49 |
| perfume | q.s. |

### Example 3

This Example also illustrates a lotion which is suitable for topical application to the scalp.

The lotion has the following formulation:

| | % w/w |
|---|---|
| Promoter No. (3) | 3 |
| propan-2-ol | 10 |
| ethanol | 87 |
| perfume | q.s. |

### Example 4

This Example also illustrates a hair tonic which is suitable for application to hair or scalp.

The hair tonic has the following formulation:

| | % w/w |
|---|---|
| Promoter No. (7) | 3 |
| ethanol | 40 |
| water | 57 |
| perfume | q.s. |

### Examples 5 to 8

The following formulations represent lotions which can be used topically in the treatment of bald or balding male or female heads.

| | % w/w | | | |
|---|---|---|---|---|
| | 5 | 6 | 7 | 8 |
| Hydroxyethyl cellulose | 0.4 | - | 0.4 | - |
| Absolute ethanol | 25 | 25 | 25 | 25 |
| Propane-1,2-diol | - | - | 38.4 | 38.4 |
| Butane-1,3-diol | 38.4 | 38.8 | - | - |
| Paramethyl benzoate | 0.2 | 0.2 | 0.2 | 0.2 |
| Promoter No. (12) | 5 | - | - | - |
| Promoter No. (13) | - | 4 | - | - |
| Promoter No. (17) | - | - | 3 | - |
| N-acetyl proline | 0.6 | 0.6 | 0.6 | 0.6 |
| Perfume | 1 | 1 | 1 | 1 |
| Water | to 100 | 100 | 100 | 100 |

### Examples 9 to 12

The following formulations represent creams which can be used in the treatment of baldness.

| | % w/w | | | |
|---|---|---|---|---|
| | 9 | 10 | 11 | 12 |
| Cetyl alcohol polyoxyethylene (10) | 4 | 4 | 4 | 4 |
| Cetyl alcohol | 4 | 4 | 4 | 4 |
| Mineral oil | 4 | 2 | - | - |
| Paraffin wax | - | 2 | 4 | - |
| Promoter No. 21 | - | - | - | 4 |
| Promoter No. 22 | 2 | - | - | - |
| Promoter No. 25 | - | 2 | - | - |
| Promoter No. 28 | - | - | 2 | - |
| minoxidil | 0.5 | 0.5 | 0.5 | 0.5 |
| Triethanolamine | 0.75 | 0.75 | 0.75 | 0.75 |
| Butane-1,3-diol | 3 | 3 | 3 | 3 |
| Xanthan gum | 0.3 | 0.3 | 0.3 | 0.3 |
| Preservative | 0.4 | 0.4 | 0.4 | 0.4 |
| Perfume | q.s. | q.s. | q.s. | q.s. |
| Water | to 100 | 100 | 100 | 100 |

### Example 13

This Example illustrates a water-in-oil high internal phase emulsion containing an amine according to the invention.

The emulsion consisted of 10% by volume oily phase and 90% by weight aqueous phase.

The oily phase and the aqueous phase had the following constitution:

| | % w/w |
|---|---|
| Oily phase | |
| Sorbitan monooleate | 20 |
| Quaternium-18 hectorite | 5 |
| Liquid paraffin | 75 |

| Aqueous phase | |
|---|---|
| Promoter No. 33 | 1 |
| Xanthan gum | 1 |
| Preservative | 0.3 |
| Perfume | q.s. |
| Sodium chloride (1% w/w solution) | to 100 |

The emulsion was prepared by taking 10 parts by volume of the oily phase and to it adding slowly with stirring 90 parts by volume of the aqueous phase.

The high internal phase water-in-oil emulsion so formed can be applied topically to the scalp, to improve hair growth and regrowth.

The following examples 14 to 18 illustrate shampoos for use in bashing the hair and scalp, and for promoting hair growth on the scalp.

### Example 14

| | % w/w |
|---|---|
| Sodium lauryl ether sulphate (2 EO) [21% AD] | 41.4 |
| Lauryl dimethylamino acetic acid betaine: [30% AD] | 4 |
| Coconut fatty acid diethanolamine | 1.5 |
| Oleyl triethoxy phosphate (BRIPHOS 03D) | 1 |
| Polyglycol-polyamine condensation resin (POLYQUART H) [50% active] | 1.5 |
| Preservative, colouring matter, salt | 0.58 |
| Promoter No. 29 | 10 |
| Perfume | q.s. |
| Water | to 100 |

### Example 15

| | % w/w |
|---|---|
| Sodium lauryl ether sulphate (2 EO) [100% AD] | 12 |
| POLYMER JR400 | 2.5 |
| BRIPHOS 03D | 2.5 |
| Promoter No. 30 | 15 |
| Magnesium Sulphate | 5 |
| Perfume | q.s. |
| Water | to 100 |

### Example 16

This Example also illustrates a lotion which is suitable for topical application to the scalp.

The lotion has the following formulation:

| | % w/w |
|---|---|
| Promoter No. 35 | 5 |
| minoxidil | 1 |
| propan-2-ol | 10 |
| ethanol | 84 |

### Examples 17

This example illustrates a powder composition according to the invention which can be applied topically to the scalp.

| | % w/w |
|---|---|
| Chemically modified starch | 5 |
| Chemically modified cellulose | - |
| Boric acid | 10 |
| Zinc oxide | 5 |
| Promoter No. 36 | 3 |
| Minoxidil | 5 |
| Perfume | q.s. |
| Chalk | 10 |
| Talc | to 100 |

### Example 18

The following example illustrates a lotion according to the invention which can be applied topically to the scalp to prevent hair loss and stimulate hair regrowth.

| | % w/w |
|---|---|
| Promoter No. (22) | 7 |
| glucaro-1,4-lactone | 2 |
| ethanol | 16 |
| citric acid | 1.05 |
| water | to 100 |
| pH adjusted to 4.2 with sodium hydroxide | |

### Examples 19 & 20

These examples illustrate hair tonics which are suitable for application to the hair and scalp.
The hair tonics had the following formulation:

| | % w/w | |
|---|---|---|
| | 19 | 20 |
| Promoter No. (2) | - | 2 |
| Promoter No. (3) | 2 | - |
| glucaro-1,5-lactam | 3 | 3 |
| ethanol | 50 | 50 |
| water | 45 | 45 |

### Example 21

This example illustrates a shampoo which is suitable for topical application to hair in order to cleanse it, at the same time delivering an inhibitor to the scalp to enhance hair growth or regrowth.

The shampoo had the following formulation:

| | % w/w |
|---|---|
| Triethanolamine lauryl sulphate | 16.8 |
| Coconut diethanolamide | 3.0 |
| Hydroxypropylmethylcellulose (1) | 0.25 |
| Corn syrup (80% solids) (2) | 20.5 |
| Dimethylpolysiloxane (3) | 1.0 |
| Cationic cellulose (4) | 0.5 |
| Ethyl alcohol (SDA 40) | 9.0 |
| Vinyl carboxy polymer (5) | 0.75 |
| Promoter No. 41 | 8 |
| Perfume, colour, preservative | q.s. |
| Water | to 100 |
| Acid or base to pH: | 6.5 |

| | |
|---|---|
| 1 - Methocel E4M (Dow Chemical) | |
| 2 - 42 Dextrose equivalent (Staley 1300) | |
| 3 - 60,000 centistokes (Viscasil, GEC) | |
| 4 - Polymer JR 400 | |
| 5 - Carbopol 941 (BF Goodrich) | |

### Example 22

This example illustrates a shampoo in accordance with the invention.

The shampoo had the following formulation:

| | %w/w |
|---|---|
| Sodium lauryl ether sulphate (3EO) | 10 |
| Pearlising agent | 4 |
| Betaine | 2 |
| Cationic polymer | 0.2 |
| Promoter No. (2) | 0.5 |
| minor ingredients | 4 |
| water | to 100 |
| pH value 6 to 7 | |

- viscosity:: 3500 to 4000 mPa·s (cps) (Brookfield Spindle No. 3 at 10 rpm 25°C)

### Example 23

This example illustrates a shampoo in accordance with this invention.

The shampoo had the following formulation:

| | %w/w |
|---|---|
| Sodium lauryl ether sulphate [3EO] (70%AD) | 20 |
| Pearlising agent | 2 |
| Betaine | 6 |
| Butyl glutamine | 1 |
| Silicone emulsion | 1 |
| Cationic polymer | 0.1 |
| D-panthenol | 0.4 |
| Carbopol | 0.4 |
| Sodium chloride | 2.5 |
| Minor ingredients | 8.5 |
| water | to 100 |
| pH value 6.5 | |

- viscosity:: 5000 mPa·s (cps) (Brookfield Spindle No. 3 at 10 rpm, 25°C)

## Claims

1. A use to induce, maintain or increase hair growth, in a method of cosmetic treatment of the mammalian body comprising topically applying to the body surface where hair growth is desired a composition comprising:
i. an effective amount of from 0.001 to 99% by weight of glutamic acid derivatives having the structure (1): 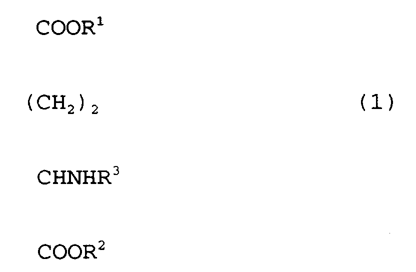 where R¹ and R² are each chosen from:
(i) H⁺,
(ii) alkali metal cations chosen from Na⁺, K⁺ and Li⁺,
(iii) NH₄⁺ or alkanolammonium ions, and
iv) CₓH_{y}-.
the R¹ group and the R² group being the same or different;
and where R³ is chosen from:
(i) H-,
(ii) C_{X}H_{y}-, and
(iii) CₓH_{y}CO-;
where x is an integer of from 1 to 22, and
y is an integer of from 3 to 45;
provided that at least one of
the R¹ and R² groups is C_{X}H_{Y}-;
and mixtures of said glutamic acid derivatives; and
ii. from 1 to 99.99% by weight of a cosmetically acceptable vehicle for the hair growth promoter.

2. A use according to claim 1, wherein the hair growth promoter is L-glutamic acid γ-n-octyl ester.

3. A use according to claim 1 or claim 2 in which the hair growth promoter forms from 0.01 to 20% by weight of the composition.

4. A use according to any one of claims 1 to 3 in which the composition further comprises an activity enhancer.

5. A use according to claim 4 in which the activity enhancer is a hair growth stimulant.

6. A use according to claim 5 in which the hair growth stimulant is minoxidil.

7. A use according to claim 4 in which the activity enhancer is a penetration enhancer.

8. A use according to claim 4 in which the activity enhancer is a cationic polymer.

9. A use according to any preceding claim, in which the composition further comprises a surface active agent.

10. A use according to any preceding claim in which the composition has a pH value in the range from 2 to less than 7.

11. A use according to any preceding claim in which the composition is a shampoo or hair conditioner.

## Patentansprüche

1. Eine Anwendung zur Einleitung, Aufrechterhaltung und Erhöhung des Haarwachstums in einem Verfahren der kosmetischen Behandlung des Säugetierkörpers, umfassend das örtliche Aufbringen einer Zusammensetzung auf die Körperoberfläche, wo Haarwachstum gewünscht wird, umfassend:
i. Eine wirksame Menge im Bereich von 0,001 bis 99 Gewichtsprozent Glutaminsäure-Derivaten der nachstehenden Struktur (1): 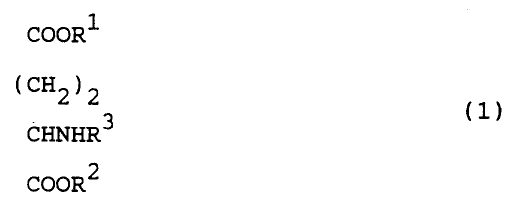 worin jeder der Reste R¹ und R² ausgewählt ist aus:
(i) H⁺,
(ii) Alkalimetallkationen, ausgewählt aus Na⁺, K⁺ und Li⁺,
(iii) NH₄⁺ oder Alkanolammoniumionen, und
(iv) CₓH_{y}-;
wobei die R¹-Gruppe und die R²-Gruppe gleich oder verschieden sind;
und worin der Rest R³ ausgewählt ist aus:
(i) H-,
(ii) CₓH_{y}-, und
(iii) CₓH_{y}CO-;
worin x eine ganze Zahl mit einem Wert von 1 bis 22 bedeutet, und
y eine ganze Zahl mit einem Wert von 3 bis 45 ist; vorausgesetzt, daß zumindest eine der Gruppen R¹ und R² CₓH_{y}- ist;
und Mischungen der genannten Glutaminsäure-Derivate; und
ii. von 1 bis 99,99 Gewichtsprozent eines kosmetisch verträglichen Trägers für den Haarwachstum-Förderer.

2. Eine Anwendung gemäß Anspruch 1, worin der Haarwachstum-Förderer L-Glutaminsäure-γ-n-octylester ist.

3. Eine Anwendung gemäß Anspruch 1 oder 2, worin der Haarwachstum-Förderer von 0,01 bis 20 Gewichtsprozent der Zusammensetzung ausmacht.

4. Eine Anwendung gemäß einem der Ansprüche 1 bis 3, in welchen die Zusammensetzung ferner einen Aktivitätserhöher enthält.

5. Eine Anwendung gemäß Anspruch 4, in welcher der Aktivitätserhöher ein Haarwachstum-Stimulans ist.

6. Eine Anwendung gemäß Anspruch 5, in welcher das Haarwachstum-Stimulans Minoxidil ist.

7. Eine Anwendung gemäß Anspruch 4, in welcher der Aktivitätserhöher ein Penetrationserhöher ist.

8. Eine Anwendung gemäß Anspruch 4, in welcher der Aktivitätserhöher ein kationisches Polymeres ist.

9. Eine Anwendung gemäß einem der vorstehenden Ansprüche, in welcher die Zusammensetzung ferner ein oberflächenaktives Mittel enthält.

10. Eine Anwendung gemäß einem der vorstehenden Ansprüche, in welcher die Zusammensetzung einen pH-Wert im Bereich von 2 bis weniger als 7 aufweist.

11. Eine Anwendung gemäß einem der vorstehenden Ansprüche, in welcher die Zusammensetzung ein Shampoo oder ein Haarkonditioniermittel ist.

## Revendications

1. Utilisation d'un traitement cosmétique du corps d'un mammifère pour induire, maintenir ou augmenter la croissance capillaire, dont le procédé comprend l'application locale à la surface corporelle où la croissance capillaire est souhaitée d'une composition comprenant :
i. une quantité efficace de 0,001 à 99% en poids de dérivés d'acide glutamique répondant à la structure (1) : 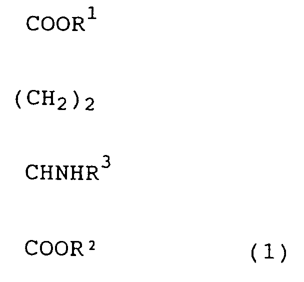 dans laquelle R¹ et R² sont chacun choisis parmi :
(i) H⁺
(ii) les cations de métaux alcalins choisis parmi Na⁺, K⁺ et Li⁺,
(iii) NH₄⁺ ou les ions alcanolammonium et
(iv) CₓH_{y}- ;
le groupe R¹ et le groupe R² étant identiques ou différents ; et dans laquelle R³ est choisi parmi :
(i) H-,
(ii) CₓH_{y}-, et
(iii) CₓH_{y}CO- ;
x étant un nombre entier de 1 à 22 et y un nombre entier de 3 à 45 ;
à la condition qu'au moins l'un des groupes R¹ et R² soit CₓH_{y}- ;
et des mélanges desdits dérivés d'acide glutamique ; et
ii. de 1 à 99,99% en poids d'un véhicule cosmétiquement acceptable pour le promoteur de croissance capillaire.

2. Utilisation selon la revendication 1, dans laquelle ledit promoteur de croissance capillaire est l'ester γ-n-octylique d'acide L-glutamique.

3. Utilisation selon la revendication 1 ou 2, dans laquelle le promoteur de croissance capillaire forme de 0,01 à 20% en poids de la composition.

4. Utilisation selon l'une quelconque des revendications 1 à 3, dans laquelle la composition comprend en outre un rehausseur d'activité.

5. Utilisation selon la revendication 4, dans laquelle le rehausseur d'activité est un stimulant de la croissance capillaire.

6. Utilisation selon la revendication 5, dans laquelle le stimulant de la croissance capillaire est le minoxidil.

7. Utilisation selon la revendication 4, dans laquelle le rehausseur d'activité est un rehausseur de la pénétration.

8. Utilisation selon la revendication 4, dans laquelle le rehausseur d'activité est un polymère cationique.

9. Utilisation selon l'une quelconque des revendications précédentes, dans laquelle la composition comprend en outre un agent tensioactif.

10. Utilisation selon l'une quelconque des revendications précédentes, dans laquelle la composition a une valeur de pH dans la gamme de 2 à moins de 7.

11. Utilisation selon l'une quelconque des revendications précédentes, dans laquelle la composition est un shampooing ou un conditionneur capillaire.
